# EUROPEAN PATENT APPLICATION

(11) **EP 1 743 672 A1**
(43) Date of publication of application: **17.01.2007**
(21) Application number: 05425505.4
(22) Date of filing: 12.07.2005
(51) Int. Cl.: A61M 16/08, A61M 16/10

(54) **Flexible duct with devices for heating the conveyed stream**

(71) Applicant: Deas S.R.L., Province of Ravenna 48014 Castel Bolognese (IT)
(72) Inventor: Scardovi, Domenico, 48014 Castel Bolognese (Ravenna) (IT)
(74) Representative: Modiano, Micaela Nadia

(57) **Abstract**

A flexible duct (1) with devices for heating the conveyed stream, which comprises a flexible tubular body (2), on the outer surface of which a wirelike stiffening element (3) is distributed substantially in a helical configuration. At least one heating filament (4) is interposed between successive starts of the helix constituted by the coiled wirelike element (3), is arranged on the outer surface of the tubular body (2) likewise substantially in a helical configuration, and is connected, at one of the ends of the duct (1), to an electrical power supply.

## Description

The present invention relates to a method for manufacturing a flexible duct with devices for heating the conveyed stream.

Respiration ducts or tubes that administer air to a patient must have a constant internal passage section and therefore must be non-deformable, because any compression thereof would entail a reduction of the air stream sent to the patient, altering the ventilation parameters suitable to ensure adequate ventilation.

This drawback has been obviated by distributing in a helical arrangement, on the outer surface of appropriate tubular bodies, stiffening ridges (normally termed spirals due to their arrangement on the ducts), which prevent the compression of the duct in normal operating conditions.

Some recent solutions provide for the insertion of the heating devices in the stiffening ridges (spirals) so that they also constitute the electrical insulation for said heating devices.

However, this solution entails difficult wiring of the heating devices, which in order to be connected to the power supply must be extracted from the ridge.

Since such ridge is made of rigid material, it is difficult to open it in order to extract the heating devices without damaging them, said devices being generally constituted by thin metal wires.

Moreover, the arrangement of the heating filaments only inside the rigid ridges does not allow uniform distribution of the heat over the entire surface of the duct, with a consequent reduced thermal conditioning efficiency and with the formation of internal condensation, which is not acceptable, since it can be potentially dangerous (risk of bacterial proliferation and inhalation).

The aim of the present invention is to obviate the cited shortcomings and meet the mentioned requirements, i.e., to provide a flexible duct with devices for heating the conveyed stream in which the heating devices can be easily connected electrically without having to cut or change the shape of the ridges.

Within this aim, an object of the present invention is to provide a structure that is simple, relatively easy to provide in practice, safe in use, effective in operation, and relatively modest in cost.

This aim and these and other objects, which will become better apparent hereinafter, are achieved by the present flexible duct with devices for heating the conveyed stream, of the type that comprises a flexible tubular body, on the outer surface of which a wirelike stiffening element is distributed substantially in a helical configuration, characterized in that at least one heating filament is interposed between successive starts of said helix constituted by the coiled wirelike element, is arranged on the outer surface of the tubular body likewise substantially in a helical configuration, and is connected, at one of the ends of said duct, to an electrical power supply.

Further characteristics and advantages of the invention will become better apparent and evident from the detailed description that follows of a preferred but not exclusive embodiment of a flexible duct with devices for heating the conveyed stream, illustrated by way of non-limiting example in the accompanying drawings, wherein:
Figure 1 is a partially sectional side view of a possible embodiment of a flexible duct according to the invention;
Figure 2 is a partially schematic side view of a first possible embodiment of a portion of a flexible duct according to the invention;
Figure 3 is a partially schematic side view of a second possible embodiment of a portion of a flexible duct according to the invention;
Figure 4 is a partially schematic side view of a third possible embodiment of a portion of a flexible duct according to the invention;
Figure 5 is a partially schematic side view of a fourth possible embodiment of a portion of a flexible duct according to the invention.

With reference to the figures, the reference numeral 1 generally designates a flexible duct with devices for heating the conveyed stream.

The duct 1 comprises a flexible tubular body 2, on the outer surface of which a wirelike stiffening element 3 is distributed substantially in a helical configuration: the wirelike element 3 is generally made of polymeric material having good rigidity, which is optionally permanently deformable in order to be able to stably give the duct 1 the chosen shape. It should be noted that it is also possible to use wirelike elements 3 provided with a core made of metal wire and sheathed (for example) with polymeric material. The proportions and the pitch of the helix along which the element 3 is wound onto the body 2 depend on the rigidity of said element 3 and on the rigidity required of the duct 1 depending on the specific application. By moving the turns closer with a particularly rigid element 3, the resulting duct 1 loses most of the flexibility of the body 2; with widely spaced turns and a more deformable element 3, it is instead possible to obtain highly flexible ducts 1.

Two heating filaments 4, connected to an electrical power supply, are arranged in a helical configuration on the outer surface of the tubular body interposed between two contiguous successive starts of the helix constituted by the coiled wirelike element 3.

The connection of the heating filaments 4 to the electric power supply generally occurs at one of the ends of the duct 1, while at the opposite end the filaments 4 are short-circuited, i.e., are mutually connected electrically.

The heating filaments 4 are arranged substantially parallel to each other and are generally parallel to the stiffening helix (constituted by the wirelike element 3).

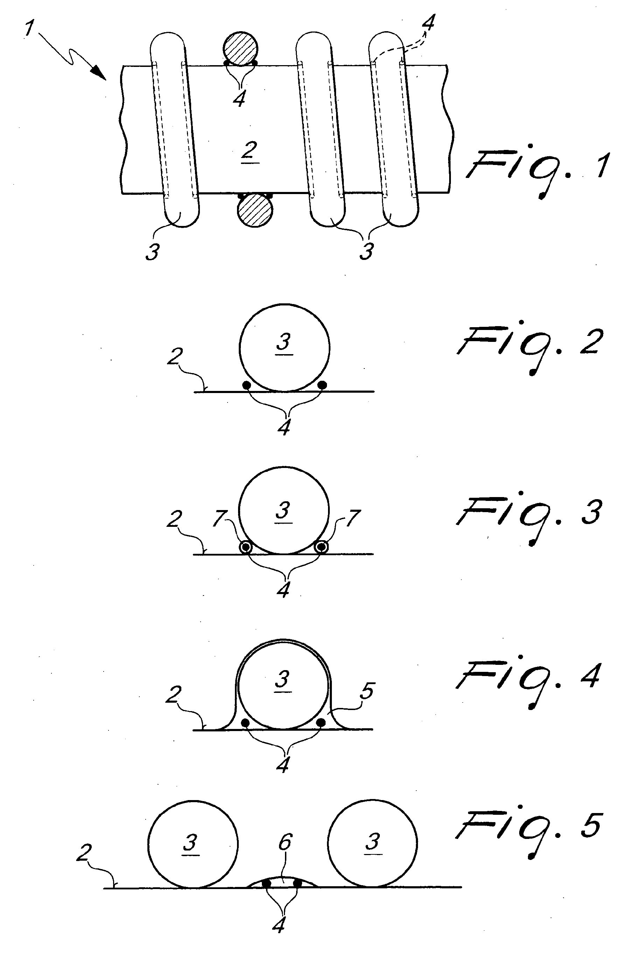

According to the constructive solutions described in Figures 2, 3 and 4, a first heating filament 4 is arranged, on the outer surface of the tubular body 2, upstream of the wirelike element with respect to the start of the stiffening helix and the second filament 4 is arranged downstream thereof: the purpose is to keep the pair of filaments 4 proximate to the wirelike element 3 so that the wirelike element protects them against contact with external elements and against impacts.

In particular, in the embodiment shown in Figure 4, it can be seen that the wirelike element 3 and the filaments 4 that lie proximate thereto are sheathed with a layer of polymeric material 5, which acts both as electrical insulation and as mechanical protection; optionally, the layer 5 can also help to increase the stiffness of the duct 1.

The embodiment of Figure 5 instead shows that the two heating filaments 4, which are mutually parallel and proximate, are arranged on the outer surface of the tubular body 2 so as to be interposed between two successive starts of the stiffening helix constituted by the wirelike element 3.

In this case, the filaments 4 and the area of the outer surface of the tubular body that is comprised between them and lies proximate thereto are covered with a layer of polymeric material 6: the function performed by such layer 6, in this case also, is to insulate electrically the two filaments 4 and protect them against externally-originating mechanical actions.

It should be noted that the heating filaments 4 are conductors with high electrical resistance which are heated by means of the Joule effect.

According to the embodiment of Figure 3, it can be seen that advantageously the heating filaments 4 can comprise a respective insulating covering sheath.

It should be noted that depending on the mechanical characteristics and dimensions of the element 3 it is possible to obtain a duct 1 having the chosen rigidity. The arrangement on the outer surface of the tubular body 2 of the filaments 4 instead allows to heat the body 2, with consequent heating of the fluid conveyed thereby, which is air in the specific case. In all of the proposed embodiments, it can be noted that advantageously the filaments 4 are always protected against impacts with external objects by the presence of the element 3 or by the layers 5 and 6 that can cover them.

It has thus been shown that the invention achieves the proposed aim and object.

The invention thus conceived is susceptible of numerous modifications and variations, all of which are within the scope of the appended claims.

All the details may furthermore be replaced with other technically equivalent ones.

In the described embodiments, individual characteristics, given in relation to specific examples, may actually be interchanged with other different characteristics that exist in other embodiments.

Moreover, it should be noted that anything found to be already known during the patenting process is understood not to be claimed and to be the subject of a disclaimer.

In practice, the materials used, as well as the shapes and the dimensions, may be any according to the requirements without thereby abandoning the scope of the protection of the claims that follow.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A flexible duct with devices for heating the conveyed stream, of the type that comprises a flexible tubular body (2), on the outer surface of which a wirelike stiffening element (3) is distributed substantially in a helical configuration, **characterized in that** at least one heating filament (4) is interposed between successive starts of said helix constituted by the coiled wirelike element (3), is arranged on the outer surface of the tubular body (2) likewise substantially in a helical configuration, and is connected, at one of the ends of said duct (1), to an electrical power supply.

2. The duct according to claim 1, **characterized in that** said heating filaments (4) are two, are arranged substantially parallel to each other, have mutually electrically connected ends, and initial ends connected to two terminals of the electric power supply unit.

3. The duct according to claims 1 and 2, **characterized in that** a first heating filament (4) is arranged, on the outer surface of said tubular body (2), upstream of said wirelike element (3) with respect to said start of the stiffening helix and the second heating filament (4) is arranged downstream thereof.

4. The duct according to one or more of the preceding claims, **characterized in that** said heating filaments (4) are parallel and proximate to said wirelike element (3).

5. The duct according to one or more of the preceding claims, **characterized in that** said wirelike element (3) and said heating filaments (4) that lie proximate thereto are covered by a layer (5) of polymeric material.

6. The duct according to claims 1 and 2, **characterized in that** the two heating filaments (4) are mutually parallel and proximate, being arranged on the outer surface of said tubular body (2) so as to be interposed between two successive starts of said stiffening helix constituted by the wirelike element (3).

7. The duct according to claim 6, **characterized in that** said heating filaments (4) and the area of the outer surface of said tubular body (2) comprised between them and arranged proximate thereto are covered with a layer (6) of polymeric material.

8. The duct according to one or more of the preceding claims, **characterized in that** said heating filaments (4) are electrical cables with high electrical resistance, which are heated by means of the Joule effect.

9. The duct according to one or more of the preceding claims, **characterized in that** said heating filaments (4) comprise a respective insulating covering sheath (7).
